# EUROPEAN PATENT APPLICATION

(11) **EP 1 348 431 A1**
(43) Date of publication of application: **01.10.2003**
(21) Application number: 03004629.6
(22) Date of filing: 03.03.2003
(51) Int. Cl.: A61K 31/335, A61K 9/16, A61K 9/50, A61K 9/51, A61K 47/42

(54) **Process for producing nanoparticles of paclitaxel and albumin**

(30) Priority: 29.03.2002 IT MI20020681
(71) Applicant: ACS DOBFAR S.p.A., 20067 Tribiano (Milano) (IT)
(72) Inventor: Zenoni, Maurizio, 20067 Paullo (MI) (IT); Maschio, Simone, 00199 Rome (IT)
(74) Representative: Frignoli, Luigi

(57) **Abstract**

A process for producing nanoparticles of paclitaxel and albumin having antitumor properties, by which a mixture obtained by adding paclitaxel in powder form to an aqueous solution of albumin with chloroform is subjected to high pressure homogenization treatment.

## Description

The present invention relates to a process for producing nanoparticles of paclitaxel and albumin, usable for obtaining antitumor compositions.

Paclitaxel is a natural substance well known in literature, with important antitumor activity: its poor water solubility makes it difficult to administer to man, for which reason various systems have been developed to render it injectable.

According to one of these systems, paclitaxel is combined with human serum albumin (HSA) which is biocompatible and has considerable capacity to bind to the paclitaxel and form injectable emulsions therewith by known ultrasonication, high pressure homogenization and microfluidization techniques (Allemann et al., Eur. J. Pharm. Biopharm. 39 (5), 173-191 (1993)).

On the basis of these elements and by using the aforestated ultrasonication and high pressure homogenization techniques, the American company VivoRx Pharmaceuticals Inc. has developed the formulation CAPXOL^{(R)} containing paclitaxel and HSA.

In US 5439686, US 5498421, US 5560933 and the corresponding WO 94/18954, VivoRx claims microparticles of paclitaxel and HSA prepared using ultrasonication techniques, to give particles of mean size (MPS) < 10 microns. The preparation methods described in these patents cannot be used on an industrial scale, and moreover the microparticles thus obtained have too high an MPS, which makes them unsuitable and unusable for administration to patients.

This was well known to the said VivoRx, which then in US 5916596 and US 6096331 and in WO 98/14174 and WO 99/00113 described and claimed sterile nanoemulsions of paclitaxel and HSA obtained by reconstituting with sterile aqueous 0.9% NaCl solution lyophilized powders with MPS < 0.2 microns. These nanoemulsions, which are obtained using high pressure homogenization, as described in the cited patents, are stated to have high stability, where the term "stability" means both that the MPS is constant with time and that nanoparticle precipitation is absent (US 6096331, Ex. 11).

According to the teachings of the aforecited VivoRx patents (see Examples 1, 5 and 6 of US 5916596), a solution of paclitaxel and an aqueous solution containing HSA are firstly prepared separately, then these phases are mixed together and the mixture so obtained is subjected to homogenization treatment at high pressure between 9000 and 40000 psi at room temperature (between 0°C and +40°C).

After evaporating the solvents and filtering through a sterile filter (0.22 microns), this mixture is frozen between -20° C and -80°C and is finally lyophilized by heating at a temperature between +20°C and +35°C to give a powder usable for preparing injectable formulations having antitumor properties.

Two separate phases (one containing paclitaxel and the other containing HSA) must therefore be prepared, to be then mixed together before their high pressure homogenization. This requires the use of at least two separate reactors and the preparation of two separate solutions with relative mixing, all to be effected under sterile conditions, involving high plant costs, lengthy times for completing the mixing operations and the need for rotavapor evaporation of the solvents (at the end of homogenization treatment) followed by filtration through a sterile filter, with consequent low overall yields.

The main object of the present invention is therefore to provide a process for producing sterile lyophilized powder of nanoparticles of paclitaxel and HSA, which requires the use of a single reactor for forming the liquid mixture containing paclitaxel and HSA to be subjected to homogenization treatment, and which can be completed in a very short time at lower cost than that of the known art.

These and further objects are attained by a process by which an aqueous mixture containing paclitaxel and albumin at a temperature between 0°C and 40°C is subjected to homogenization treatment at high pressure between 9000 and 40000 psi, to give a nanoemulsion which is frozen between -20°C and -80°C and is finally lyophilized by heating at a temperature between +20°C and +35°C, wherein said aqueous mixture is obtained under sterile conditions by dissolving said albumin in sterile water to a concentration between 2% and 3% (w/v), then adding to said albumin solution between 2% and 4% (v/v) of chloroform and then paclitaxel in sterile powder form in a quantity between 5.4% and 20.0% by weight on the weight of the albumin present in the solution. The quantity of paclitaxel in sterile powder form added to the liquid mixture is preferably between 5.6% and 19.4% by weight on the albumin.

It is important to note that the use of paclitaxel in sterile powder form in the process not only greatly simplifies the plant itself and the process compared with the known art and enables the time required to complete the mixing of the various components before the homogenization treatment to be considerably shortened, but also enables better final yields to be obtained and simplifies the conditions to be observed in order to obtain the desired sterile lyophilized powders.

By operating in the aforestated manner powders formed by mixtures of nanoparticles of paclitaxel and HSA are obtained which are totally similar or equal to those obtainable by the more complex, laborious and costly methods described in the aforestated prior patents.

From these mixtures, when processed with an Avestin homogenizer within the pressure range recommended in US 5916596, nanoemulsions at pH=6.7 are obtained which, when evaporated in a rotavapor as reported in the said patent, provide nanoemulsions with MPS of about 0.2 microns (increase of MPS > 0.02 microns after evaporation) which are poorly stable in their formulations in injectable physiological solutions (increase in MPS of about 0.05 microns and tendency to sediment in about 12 hours) and difficult to filter through 0.22 microns filters for their sterilization, these filters being easily clogged and reducing the paclitaxel yield to a very low value (down to or less than 30%).

The stability (evaluated in accordance with the teachings of Example 11 of US 6096331) of the products prepared both by the method of the present invention as hereinbefore defined and in accordance with the prior patents, when lyophilized and reconstituted as reported in US 5916596 and US 6096331, is acceptable but never exceeds 24 hours.

It has been surprisingly found that if at least one biocompatible acid is added to the liquid mixture containing the HSA (before the paclitaxel in powder form is added to it) in a quantity sufficient to bring to between 5.4 and 5.8 (preferably between 5.5 and 5.7) the pH of a reconstituted aqueous injectable mixture of the nanoparticles in powder form, the stability of the lyophilized and reconstituted mixture in injectable form considerably increases, beyond 24 hours.

The addition of the aforesaid acid or also forms part of the present invention.

Preferably, said acid is chosen from the group consisting of HCl, citric acid, phosphoric acid, acetic acid, biocompatible organic and inorganic acids, but citric acid is the most preferred one.

To clarify the characteristics of the present invention, some nonlimiting examples of its implementation will now be described, some with liquid mixtures at physiological pH and some acidified to highlight the differences consequent on the use of the acids.

### EXAMPLE 1

### Preparation of formulation at pH 6.7

An injectable aqueous 20% (w/v) HSA solution in accordance with FDA specifications (pH=6.9±0.5) is diluted to 3% (w/v) with sterile demineralized water.

41.4 ml of said solution are mixed under energetic agitation with 1.25 ml of sterile chloroform and with 73.6 mg (5.9% by weight on the weight of the albumin in the solution) of sterile paclitaxel (titre > 99%) in powder form, then the mixture is processed in a high pressure homogenizer (suitably sterilized) until a nanoemulsion (MPS about 0,2 microns) is obtained, this being evaporated under vacuum to remove the solvent, frozen and lyophilized under sterile conditions for 48 hours.

The powder obtained, containing 4.60 % (w/w) of paclitaxel, is reconstituted with an aqueous 0.9% NaCl solution to a paclitaxel concentration of 2 mg/ml. The formulation obtained has an MPS of 0.486 microns, pH=6.7, and a stability < 12 hours.

The product obtained has the same characteristics as that prepared by the method used in Example 1 of US 5916596.

### EXAMPLE 2

### Preparation of formulation at pH 6.7

An injectable aqueous 25% (w/v) HSA solution in accordance with FDA specifications (pH=6.9±0.5) is diluted to 2% (w/v) with sterile demineralized water.

49.0 ml of said solution are mixed with 1.0 ml of sterile chloroform and with 72.5 mg (7.4% to albumin) of sterile paclitaxel (titre > 99%) in powder form, then the mixture is processed in a high pressure homogenizer (suitably sterilized) until a nanoemulsion (MPS about 0,2 microns) is obtained, this being evaporated under vacuum to remove the solvents, filtered through a sterile filter (0.2 microns), frozen and lyophilized under sterile conditions for 48 hours.

The powder obtained, containing 0.60% (w/w) of paclitaxel, is reconstituted with an aqueous 0.9% NaCl solution to a paclitaxel concentration of 2 mg/ml. The formulation obtained has an MPS of 0,25 microns, pH=6.7, and a stability < 12 hours.

### EXAMPLE 3

### Preparation of formulation at pH 6.7

An injectable aqueous 20% (w/v) HSA solution in accordance with FDA specifications (pH=6.9±0.5) is diluted to 3% (w/v) with sterile demineralized water.

46.7 ml of said solution are mixed with 1.40 ml of sterile CHCl₃ and with 108.5 mg (7.7% to albumin) of sterile paclitaxel (titre > 99%) in powder form, then the mixture is processed in a high pressure homogenizer (suitably sterilized) until a nanoemulsion (MPS about 0.2 microns) is obtained, this being evaporated under vacuum to remove the solvents, filtered through a sterile filter (0.2 microns), frozen and lyophilized under sterile conditions for 48 hours.

The powder obtained, containing 0.77 % (w/w) of paclitaxel, is reconstituted with an aqueous 0.9% NaCl solution to a paclitaxel concentration of 2 mg/ml. The formulation obtained has an MPS of 0,12 microns, pH=6.7, and a stability < 12 hours.

As already stated in the initial descriptive part, the filtration resulted in a considerable loss of paclitaxel (the lyophilized powder contains 0.55% of paclitaxel instead of the 5.2% of Example 2). This enabled a formulation to be obtained with MPS < 0,2 microns.

### EXAMPLE 4

### Preparation of formulation at pH 6.7

An injectable aqueous 25% (w/v) HSA solution in accordance with FDA specifications (pH=6.9±0.5) is diluted to 3% (w/v) with sterile demineralized water.

29.1 ml of said solution are mixed with 0.90 ml of sterile CHCl₃ and 67.0 mg (7.7% to albumin) of sterile paclitaxel (titre > 99%) in powder form, then the mixture is processed in a high pressure homogenizer (suitably sterilized) until a nanoemulsion (MPS about 0.2 microns) is obtained, this being evaporated under vacuum to remove the solvents, filtered through a sterile filter (0.2 microns), frozen and lyophilized under sterile conditions for 48 hours.

The powder obtained, containing 0.70% (w/w) of paclitaxel, is reconstituted with an aqueous 0.9% NaCl solution to a paclitaxel concentration of 1.5 mg/ml. The formulation obtained has an MPS of 0.25 microns, pH=6.7, and a stability < 12 hours.

The product obtained has the same characteristics as that prepared by the method used in Example 5 of US 5916596.

### EXAMPLE 5

### Preparation of formulation at pH 6.7

An injectable aqueous 20% (w/v) HSA solution in accordance with FDA specifications (pH=6.9±0.5) is diluted to 2.5% (w/v) with sterile demineralized water and presaturated with chloroform (1% v/v).

48.5 ml of said solution are mixed with 1.0 ml of sterile CHCl₃ and with 75 mg (6.2% to albumin) of sterile paclitaxel (titre > 99%) in powder form, then the mixture is processed in a high pressure homogenizer (suitably sterilized) until a nanoemulsion (MPS about 0.2 microns) is obtained, this being evaporated under vacuum to remove the solvent, filtered through a sterile filter (0.2 microns), frozen and lyophilized under sterile conditions for 48 hours.

The powder obtained, containing 0.70 % (w/w) of paclitaxel, is reconstituted with an aqueous 0.9% NaCl solution to a paclitaxel concentration of 2.2 mg/ml. The formulation obtained has an MPS of 0.18 microns, pH=6.7, and a stability < 12 hours.

Again in this case the observations made at the end of Example 3 are valid.

### EXAMPLE 6

### Preparation of formulation at pH=5.6

An injectable aqueous 25% (w/v) HSA solution in accordance with FDA specifications (pH=6.9±0.5) is diluted to 3% (w/v) with sterile demineralized water, the pH being corrected to a value of 5.6 with 1M HCl which salifies some basic groups present in albumin.

57 ml of said solution, previously sterilized, are mixed under vigorous stirring for at least 30 minutes, with 1.40 ml of sterile chloroform and with 108 mg (6.3% to albumin) of sterile paclitaxel (titre > 99%) in powder form.

The mixture is processed in a homogenizer (suitably sterilized) at high pressure (9000-40000 psi) until a nanoemulsion (MPS < 0,2 microns) is obtained, this being rapidly frozen to -80°C and lyophilized for 55 hours under sterile conditions, while raising the temperature to +30°C.

The powder obtained, containing 4.83 % (w/w) of paclitaxel and 4% (w/w) of water, is reconstituted with an aqueous 0.9% NaCl solution to a paclitaxel concentration of 2 mg/ml. The formulation obtained has an MPS of 0.175 microns, pH=5.6, and a stability > 24 hours.

Equivalent results are obtained by using phosphoric acid instead of hydrochloric acid.

### EXAMPLE 7

### Preparation of formulation at pH=5.4

An injectable aqueous 25% (w/v) HSA solution in accordance with FDA specifications is diluted to 3% (w/v) with sterile demineralized water, the pH being corrected to a value of 5.4 with citric acid which salifies some basic groups present in albumin.

50 ml of said solution, previously sterilized, are mixed under vigorous stirring for at least 40 minutes, with 1.23 ml of sterile chloroform and with 98 mg (6.5% to albumin) of sterile paclitaxel (titre > 99%) in powder form.

The mixture is processed in a homogenizer (suitably sterilized) at high pressure (9000-40000 psi) until a nanoemulsion (MPS < 0.2 microns) is obtained, this being rapidly frozen to -30°C and lyophilized for 57 hours under sterile conditions, while raising the temperature to +35°C.

The powder obtained, containing 4.80 % (w/w) of paclitaxel and 3.8% (w/w) of water, is reconstituted with an aqueous 0.9% NaCl solution to a paclitaxel concentration of 2 mg/ml. The formulation obtained has an MPS of 0.19 microns, pH=5.4, and a stability > 24 hours.

Equivalent results are obtained by using acetic acid instead of citric acid.

### EXAMPLE 8

### Preparation of formulation at pH=5.5

An injectable aqueous 25% (w/v) HSA solution in accordance with FDA specifications is diluted to 3% (w/v) with sterile demineralized water, the pH being corrected to a value of 5.5 with sterile citric acid which salifies some basic groups present in albumin.

37 ml of said solution are mixed under vigorous stirring for at least 40 minutes, with 0.91 ml of sterile chloroform and 71 mg (6.4% to albumin) of sterile paclitaxel (titre > 99%) in powder form, after which the mixture is cooled to 5-8°C.

The mixture is processed in a homogenizer (suitably sterilised) at high pressure (9000-40000 psi) until a nanoemulsion (MPS < 0.2 microns) is obtained, this being rapidly frozen to -80°C and lyophilized for 58 hours under sterile conditions, while raising the temperature to +30°C.

The powder obtained, containing 4.70 % (w/w) of paclitaxel and 4.5% (w/w) of water, is reconstituted with an aqueous 0.9% NaCl solution to a paclitaxel concentration of 2 mg/ml. The formulation obtained has an MPS of 0.185 microns, pH=5.5, and a stability > 24 hours.

### EXAMPLE 9

### Preparation of formulation at pH 5.5

An injectable aqueous 20% (w/v) HSA solution in accordance with FDA specifications (pH=6.9±0.5) is diluted to 3% (w/v) with sterile demineralized water, the pH being corrected to a value of 5.5 with citric acid which salifies some basic groups present in albumin.

110 ml of said solution are mixed with 4.10 ml of sterile CHCl₃ and with 639 mg (19.4% to albumin) of sterile paclitaxel (titre > 99%) in powder form, then the mixture is processed in a high pressure homogenizer (suitably sterilized) until a nanoemulsion (MPS about 0.2 microns) is obtained, this being filtered through a sterile filter (0.2 microns), evaporated under vacuum to remove the solvents, frozen and lyophilized under sterile conditions for 48 hours.

The powder obtained, containing 10.8 % (w/w) of paclitaxel, is reconstituted with an aqueous 0.9% NaCl solution to a paclitaxel concentration of 2 mg/ml. The formulation obtained has an MPS of 0.15 microns and a stability > 24 hours.

## Claims

1. A process for producing a sterile lyophilized powder consisting of nanoparticles of paclitaxel and human serum albumin, by which an aqueous mixture containing paclitaxel and albumin at a temperature between 0°C and 40°C is subjected to homogenization treatment at high pressure between 9000 and 40000 psi, to give a nanoemulsion which is frozen between -20°C and -80°C and is finally lyophilized by heating to between +20°C and +35°C, **characterized in that** said aqueous mixture is obtained under sterile conditions by dissolving between 2% and 3% (w/v) of said albumin in sterile water, then adding to said albumin solution between 2% and 4% (v/v) of sterile chloroform and then paclitaxel in sterile powder form in a quantity between 5.4% and 20.0% by weight on the weight of the albumin present in the solution.

2. A process as claimed in claim 1, **characterized in that** the quantity of paclitaxel in sterile powder form added to said albumin solution is between 5.6% and 19.4% by weight on the albumin weight.

3. A process as claimed in claims 1 and 2, **characterized by** adding to said albumin solution, before adding the paclitaxel, at least one biocompatible acid in a quantity sufficient to bring to between 5.4 and 5.8 the pH of a reconstituted aqueous injectable mixture of the nanoparticles in powder form.

4. A process as claimed in claim 3, **characterized in that** the quantity of said acid is such as to bring the pH of said reconstituted aqueous solution to between 5.5 and 5.7.

5. A process as claimed in claims 3 and 4, **characterized in that** said acid is chosen from the group consisting of HCl, citric acid, phosphoric acid, acetic acid, biocompatible organic and inorganic acids.

6. A process as claimed in claim 5, **characterized in that** said acid is citric acid.
